# EUROPEAN PATENT SPECIFICATION

(11) **EP 0 782 858 B1**
(45) Date of publication and mention of the grant of the patent: **21.11.2001**
(21) Application number: 96203204.1
(22) Date of filing: 15.11.1996
(51) Int. Cl.: A61K 35/78, A23L 1/10

(54) **A growth promoting substance**
Wachstumfördernde Substanz
Substance favorisant la croissance

(30) Priority: 17.11.1995 NL 1001670; 12.01.1996 NL 1002085
(43) Date of publication of application: 09.07.1997
(73) Proprietor: van Eijsselsteijn, Danny, 6101 MB Echt (NL); van Eewijk, Wouter Paulus, 5916 NC Venlo (NL); Dost, Anna Francisca Gertruda, 6101 MB Echt (NL)
(72) Inventor: van Eijsselsteijn, Danny, 6101 MB Echt (NL)
(74) Representative: Van kan, Johan Joseph Hubert, Ir.

(56) References cited:
- FR-A- 2 655 820

## Description

The invention relates to a the use of hop for the manufacture of a powdery substance for the development of breast of the female figure. The invention furthermore relates to a powdery substance for promoting the growth of the breast of a female figure, as well as tablets and to a method for preparing a liquid substance for use in promotion the growth of the breast of a female figure, and to the composition thus obtained, which is taken orally.

Growth promoting substances and in particular substances for changing the shape of the human body are generally known, but one drawback of these substances is that they exhibit undesirable side effects, because they are composed on a base of hormones and the like. Furthermore changes of the female figure are often carried out by implanting silicone prostheses, which has generally known drawbacks and which is very costly. Now a substance having a natural base has been found, which will produce a visible and, for the person who takes the substance, tangible result after the substance has been taken for a relatively short period.

The substance according to the invention is composed in the form of a powder, from which tablets are made or which is dissolved and/or suspended in water and subsequently taken by the user. It will also be possible, of course, to market the composition in dissolved/suspended condition.

The powdery substance according to the invention is characterized in that it contains hop. Furthermore the substance may contain malt and wheat.

The action of the substance may furthermore be stimulated by adding one or more materials selected from the group consisting of rye, barley, maize, oats and yeast. In order to mask the slightly bitter taste of this substance it is recommended to add flavouring substances, in particular substances having different flavours which are known in connection with the preparation of instant pudding or the like.

Furthermore it is preferable to use the above agricultural products in their most natural form, viz. in the form in which the products such as malt, wheat, barley, rye, maize and oats are harvested, that is, the whole grains complete with the pellicles. It is furthermore preferred for the hop to be brewer's hop and the yeast to be baker's yeast.

Experiments have shown that 1000 g of substance is preferably composed of 100-300 g each of hop, malt and wheat, to which 150-250 g of rye may be added, with the balance of said 1000 g consisting of the other components, including substances which are neutral to the growth process, such as flavouring substances.

Preferably about 2 level teaspoonfuls (about 5 g) of the powdery substance are mixed with about 100 ml of water, which mixture is taken after stirring. When used on a daily basis a positive effect may be perceived after one month already.

The invention will be explained in more detail below by means of the following examples.

### Example 1

200 g of hop, 200 g of malt and 200 g of wheat are mixed, whereby the cereals are whole grains, that is, complete with the pellicles, to which 200 g of rye are added. Then this mixture is furthermore mixed with 40 g of baker's yeast, 80 g of oats and 80 g of maize.

After the materials have been mixed to form a substantially homogeneous mixture, 5 g of the substance thus formed are suspended in 100 ml of water and stirred properly. Then the composition is taken by the user. After the substance has been taken once a day for one month, a positive result may already be expected with regard to the breast development.

### Example 2

The composition of example 1 is prepared, whereby 160 g of oats and maize are substituted for 50 g of oats, 50 g of maize and 60 g of flavouring substance, viz. instant pudding powder with a raspberry flavour.

### Example 3

140 g of rye, 140 g of wheat, 140 g of malt, 140 g of oats, 140 g of maize and 300 g of barley are mixed, to which mixture 200 g of hop are added.

After intensive mixing of these components to provide a homogeneous mixture weighing 1200 g, 1200 tablets, each having a weight of 1 g, are formed from said mixture.

Said tablets will produce an intended result after being taken in an amount of 10 tablets per day for a period of 60 days.

## Claims

1. The use of hop for the manufacture of a powdery substance for promoting the growth of the breast of the female figure.

2. The use of hop according to claim 1, **characterized in that** said substance further contains malt and wheat.

3. The use of hop according to claim 1, **characterized in that** said substance contains rye.

4. The use of hop according to any one of the preceding claims, **characterized in that** said substance contains barley.

5. The use of hop according to any one of the preceding claims, **characterized in that** said substance contains maize.

6. The use of hop according to any one of the preceding claims, **characterized in that** said substance contains oats.

7. The use of hop according to any one of the preceding claims, **characterized in that** said substance contains yeast.

8. The use of hop of according to any one of the preceding claims, **characterized in that** said substance contains flavouring substances.

9. A powdery substance for promoting the growth of the breast of a female figure, **characterized in that** 1000 g of said substance contain 100-300 g each of hop, malt and wheat.

10. A powdery substance according to claim 9, **characterized in that** 1000 g of said substance contain 150-250 g each of hop, malt and wheat.

11. A powdery substance according to claims 9-10, **characterized in that** said substance contains 100-300 g of rye.

12. A powdery substance according to claims 9-11, **characterized in that** the balance of said 1000 g consists of barley, oats, maize and/or yeast and/or flavouring substances.

13. Tablets made with the powdery substance as defined in claims 9-12 as a base.

14. Tablets according to claim 13, **characterized in that** said tablets have a weight of 0.5-2 g.

15. A method for preparing a liquid substance for use in promoting the growth of the breast of a female figure, **characterized in that** water is mixed with the powdery substance according to the present claims 9-12.

16. A method according to claim 15, **characterized in that** 2-10 g of said substance is mixed with 100 ml of water.

17. A liquid substance for promoting the growth of the breast of a female figure, **characterized in that** the substance is prepared in accordance with a method as described in claims 15-16.

## Patentansprüche

1. Verwendung von Hopfen zur Herstellung einer pulvrigen Substanz für die Wachstumsförderung der weiblichen Brust.

2. Verwendung von Hopfen nach Anspruch 1,
**dadurch gekennzeichnet, dass** die Substanz zusätzlich Malz und Weizen enthält.

3. Verwendung von Hopfen nach Anspruch 1,
**dadurch gekennzeichnet, dass** die Substanz Roggen enthält.

4. Verwendung von Hopfen nach einem der vorhergehenden Ansprüche,
**dadurch gekennzeichnet, dass** die Substanz Gerste enthält.

5. Verwendung von Hopfen nach einem der vorhergehenden Ansprüche,
**dadurch gekennzeichnet, dass** die Substanz Mais enthält.

6. Verwendung von Hopfen nach einem der vorhergehenden Ansprüche,
**dadurch gekennzeichnet, dass** die Substanz Hafer enthält.

7. Verwendung von Hopfen nach einem der vorhergehenden Ansprüche,
**dadurch gekennzeichnet, dass** die Substanz Hefe enthält.

8. Verwendung von Hopfen nach einem der vorhergehenden Ansprüche,
**dadurch gekennzeichnet, dass** die Substanz aromatische Geschmacksstoffe enthält.

9. Pulvrige Substanz zur Wachstumsförderung der weiblichen Brust,
**dadurch gekennzeichnet, dass** in 1000 g der Substanz jeweils 100 bis 300 g Hopfen, Malz und Weizen enthalten sind.

10. Pulvrige Substanz nach Anspruch 9,
**dadurch gekennzeichnet, dass** in 1000 g der Substanz jeweils 150 bis 250 g Hopfen, Malz und Weizen enthalten sind.

11. Pulvrige Substanz nach Anspruch 9 oder 10,
**dadurch gekennzeichnet, dass** die Substanz 100 bis 300 g Roggen enthält.

12. Pulvrige Substanz nach einem der Ansprüche 9 bis 11,
**dadurch gekennzeichnet, dass** der Rest zu den 1000 g aus Gerste, Hafer, Mais und/oder Hefe und/oder aromatischen Geschmacksstoffen besteht.

13. Tabletten, hergestellt aus der in Anspruch 9 bis 12 definierten pulvrigen Substanz als Grundstoff.

14. Tabletten nach Anspruch 13,
**dadurch gekennzeichnet, dass** die Tabletten ein Gewicht von 0,5 bis 2 g aufweisen.

15. Verfahren zur Herstellung einer flüssigen Substanz für die Verwendung zur Wachstumsförderung der weiblich Brust,
**dadurch gekennzeichnet, dass** Wasser mit der pulvrigen Substanz gemäß Anspruch 9 bis 12 gemischt wird.

16. Verfahren nach Anspruch 15,
**dadurch gekennzeichnet, dass** zwei bis zehn g der Substanz mit 100 ml Wasser gemischt werden.

17. Flüssige Substanz für die Wachstumsförderung der weiblichen Brust
**dadurch gekennzeichnet, dass** die Substanz gemäß einem Verfahren wie in den Ansprüchen 15 oder 16 beschrieben, hergestellt wird.

## Revendications

1. Utilisation du houblon pour la fabrication d'une substance poudreuse promouvant la croissance de la poitrine du corps féminin.

2. Utilisation du houblon selon la revendication 1, **caractérisée en ce que** ladite substance contient en outre du malt et du blé.

3. Utilisation du houblon selon la revendication 1, **caractérisée en ce que** ladite substance contient du seigle.

4. Utilisation du houblon selon l'une quelconque des revendications précédentes, **caractérisée en ce que** ladite substance contient de l'orge.

5. Utilisation du houblon selon l'une quelconque des revendications précédentes, **caractérisée en ce que** ladite substance contient du maïs.

6. Utilisation du houblon selon l'une quelconque des revendications précédentes, **caractérisée en ce que** ladite substance contient de l'avoine.

7. Utilisation du houblon selon l'une quelconque des revendications précédentes, **caractérisée en ce que** ladite substance contient de la levure.

8. Utilisation du houblon selon l'une quelconque des revendications précédentes, **caractérisée en ce que** ladite substance contient des substances aromatiques.

9. Substance poudreuse pour la promotion de la croissance de la poitrine d'un corps féminin, **caractérisée en ce que** 1 000 g de ladite substance contiennent 100 à 300 g respectivement de houblon, de malt et de blé.

10. Substance poudreuse selon la revendication 9, **caractérisée en ce que** 1 000 g de ladite substance contiennent 150 à 250 g respectivement de houblon, de malt et de blé.

11. Substance poudreuse selon les revendications 9, et 10 **caractérisée en ce que** ladite substance contient 100 à 300 g de seigle.

12. Substance poudreuse selon les revendications 9, à 11 **caractérisée en ce que** l'équilibre desdits 1 000 g consiste en de l'orge, de l'avoine, du maïs et/ou de la levure et/ou des substances aromatiques.

13. Comprimés fabriqués avec ladite substance poudreuse définie aux revendications 9 à 12 comme base.

14. Comprimés selon la revendication 13, **caractérisés en ce que** lesdits comprimés ont un poids de 0,5 à 2 g.

15. Procédé pour la préparation d'une substance liquide utilisée pour promouvoir la croissance de la poitrine d'un corps féminin **caractérisée en ce que** de l'eau est mélangée avec la substance poudreuse selon les présentes revendications 9 à 12.

16. Procédé selon la revendication 15, **caractérisée en ce que** 2 à 10 g de ladite substance sont mélangés avec 100 ml d'eau.

17. Substance liquide pour la promotion de la croissance de la poitrine d'un corps féminin **caractérisée en ce que** la substance est préparée selon un procédé comme décrit dans les revendications 15 et 16.
